# EUROPÄISCHE PATENTSCHRIFT

(11) **EP 2 752 164 B1**
(45) Veröffentlichungstag und Bekanntmachung des Hinweises auf die Patenterteilung: **07.10.2015**
(21) Anmeldenummer: 13192482.1
(22) Anmeldetag: 12.11.2013
(51) Int. Cl.: A61B 17/122

(54) **Aneurysma-Clip**
Aneurysm clip
Clip d'anévrisme

(30) Priorität: 08.01.2013 DE 102013200127
(43) Veröffentlichungstag der Anmeldung: 09.07.2014
(73) Patentinhaber: Peter Lazic GmbH, 78532 Tuttlingen (DE)
(72) Erfinder: Lazic, Daniel, 78532 Tuttlingen (DE)
(74) Vertreter: Kohler Schmid Möbus Patentanwälte

(56) Entgegenhaltungen:
- DE-A1- 3 523 031
- DE-A1- 10 309 491
- DE-A1-102009 003 273

## Beschreibung

Die Erfindung betrifft einen chirurgischen Clip, wie z.B. einen Aneurysma-Clip, gemäß Oberbegriff von Anspruch 1 oder Anspruch 3.

Ein derartiger Aneurysma-Clip ist beispielsweise durch die DE 103 09 491 A1 bekannt geworden.

Bei dem aus DE 103 09 491 A1 bekannten chirurgischen Clip ist an dem einen Clipteil eine Lagerhülse angeformt, in der das andere Clipteil mittels eines Lagerrings drehbar gelagert ist.

Aus der DE 35 23 031 A1 ist ein Clip bekannt, bei dem die beiden Clipteile auf einem Lagerstift drehbar gelagert sind.

Aus der DE 10 2004 016 859 A1 ist weiterhin ein Aneurysma-Clip mit zwei miteinander verbundenen Clipteilen bekannt, von denen das eine, erste Clipteil in eine Drehaufnahme des anderen, zweiten Clipteils eingesteckt und darin durch ein Führungsplättchen gesichert ist. Die Drehaufnahme der beiden Clipteile weist jeweils zwei bezüglich der Drehachse einander gegenüberliegende Drehlagerabschnitte auf, in denen das jeweils andere Clipteil drehbar gelagert ist. Allerdings erstrecken sich die Drehlagerabschnitte jeweils nur auf einem Winkelbereich von ca. 75°. Das Führungsplättchen wird nach dem Zusammenstecken der beiden Clipteile am zweiten Clipteil angeschweißt und verhindert so das Lösen der beiden Clipteile entgegen der Steckrichtung. Das Anschweißen des Führungsplättchens erfordert eine aufwändige Montage, bei der das verschweißte Plättchen spanend nachgearbeitet wird, um eine Gratbildung zu verhindern. Abschließend wird eine Schenkelfeder in einer zentralen Öffnung der beiden Clipteile angeordnet und mit ihren Federschenkeln an den beiden Clipteilen angeschweißt.

Schließlich ist aus der DE 10 2009 003 273 A1 noch ein Aneurysma-Clip mit zwei durch einen Drehsteckschluss miteinander verbundenen und drehbar gelagerten Clipteilen bekannt. Jedes der beiden Clipteile weist zwei bezüglich der Drehachse einander gegenüberliegende Drehlagerabschnitte aufweisen, in denen das jeweils andere Clipteil drehbar gelagert ist. Allerdings erstrecken sich die Drehlagerabschnitte jeweils nur auf einem Winkelbereich von weniger als 30°. Eine Schenkelfeder ist in einer zentralen Öffnung der beiden Clipteile angeordnet und mit ihren Federschenkeln an den beiden Clipteilen angeschweißt.

Demgegenüber ist es die Aufgabe der vorliegenden Erfindung, bei einem chirurgischen Clip der eingangs genannten Art sowohl Fertigungskosten und Montageaufwand zu reduzieren als auch die Drehlagerung der beiden Clipteile weiter zu verbessern.

Diese Aufgabe wird erfindungsgemäß durch einen chirurgischen Clip mit den Merkmalen von Anspruch 1 oder Anspruch 3 gelöst.

Die Lagerhülse bildet somit die Drehlagerung, um die sich die beiden Clipteile drehen. Die Lagerhülse kann entweder ein separates Teil sein oder mit dem Clipteil unverdrehbar steckverbunden sein. In den beiden Fällen besteht der chirurgische Clip aus vier Einzelteilen (zwei Clipteile, Lagerhülse und Schenkelfeder).

Wenn die beiden Clipteile aus schweißbarem Material, z.B. aus Titan, gebildet sind, können die beiden Federschenkel der Schenkelfeder mit den beiden Clipteilen verschweißt sein oder alternativ mit der drehfest an dem einen Clipteil befestigten Lagerhülse und mit dem anderen Clipteil verschweißt sein.

Wenn die beiden Clipteile aus nicht-schweißbarem Material, wie z.B. aus Kunststoff, insbesondere aus Polymethylmethacrylat (PMMA) oder aus röntgentransparentem Polyetheretherketon (PEEK), gebildet sind, kann der eine Federschenkel der Schenkelfeder mit der drehfest an dem einen Clipteil befestigten Lagerhülse verschweißt sein und der andere Federschenkel am anderen Clipteil angreifen, also beispielsweise den anderen Clipteil - ähnlich einer Wäscheklammer - außenseitig umklammern.

Um Quetschungen von Gewebe zwischen den einzelnen Schraubenwindungen der Schenkelfeder auszuschließen, sind alle Schraubenwindungen, also der gesamte Windungskörper der Schenkelfeder, vollständig innerhalb der Lagerhülse angeordnet.

Bei einer besonders bevorzugten Ausführungsform der Erfindung sind die beiden Clipteile durch einen Steck-Drehverschluss miteinander verbunden, bei dem die beiden Clipteile in einer Montagedrehposition axial ineinander gesteckt und durch anschließendes Verdrehen in Richtung auf die Ausgangsdrehstellung miteinander axial verriegelt sind. Der Steck-Drehverschluss ermöglicht das Ineinandersetzen der beiden Clipteile und deren Drehführung ohne zusätzliche Bauteile und ohne zusätzlichen Montageaufwand.

In einer vorteilhaften Weiterbildung dieser Ausführungsform ist vorgesehen, dass der Ringabschnitt mindestens eines der beiden Clipteile zwei bezüglich der Öffnung einander gegenüberliegende erste Ringsegmente und dazwischen jeweils ein gegenüber den ersten Ringsegmenten radial nach innen zurückversetztes zweites Ringsegment aufweist und dass mindestens das andere Clipteil eine Steckaufnahme aufweist, die durch zwei bezüglich der Öffnung einander gegenüberliegende Seitenwände mit darin vorgesehen Umfangsnuten gebildet ist, wobei in der Montagedrehposition das eine Clipteil mit seinen beiden zweiten Ringsegmenten zwischen die Seitenwände des jeweils anderen Clipteils in die Steckaufnahme gesteckt und durch anschließendes Verdrehen mit seinen ersten Ringsegmenten in die Umfangsnuten des anderen Clipteils eingreift und dadurch die beiden Clipteile miteinander entgegen der Steckrichtung verriegelt sind.

Bei einer weiteren bevorzugten Ausführungsform der Erfindung sind die beiden Clipteile durch die Lagerhülse, deren eines Ende einen Ringbund und deren anderes Ende einen nach außen umgebogenen Nietkopf aufweist, auch axial zusammengehalten.

Weitere Vorteile der Erfindung ergeben sich aus der Beschreibung, den Ansprüchen und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigen:
- Fign. 1a-1c: eine erste Ausführungsform des erfindungsgemäßen Aneurysma-Clips, der aus zwei Clipteilen und einer Lagerhülse zusammengesteckt ist, in geschlossenem Zustand in einer Draufsicht auf die eine Clipseite (Fig. 1a), in einer Schnittansicht (Fig. 1b) und in einer Draufsicht auf die in Fig. 1 a nicht sichtbare andere Clipseite (Fig. 1 c);
- Fign. 2a-2c: das in Fig. 1 gezeigte eine, erste Clipteil in einer Draufsicht auf die Clipteil-Innenseite (Fig. 2a), in einer Schnittansicht (Fig. 2b) und in einer Draufsicht auf die Clipteil-Außenseite (Fig. 2c);
- Fign. 3a, 3b: das in Fig. 1 gezeigte andere, zweite Clipteil in einer Draufsicht (Fig. 3a) und in einer Seitenansicht (Fig. 3b);
- Fig. 4: die beiden Clipteile, die für einen Drehsteckschluss in einer maximal weit geöffneten Montagedrehposition ineinander gesteckt und über die Lagerhülse drehbar gelagert sind;
- Fign. 5a-5c: eine Ausführungsform eines nicht erfindungsgemäßen Aneurysma-Clips, der aus zwei Clipteilen zusammengesteckt ist, in geschlossenem Zustand in einer Draufsicht auf die eine Clipseite (Fig. 5a), in einer Schnittansicht (Fig. 5b) und in einer Draufsicht auf die in Fig. 5a nicht sichtbare andere Clipseite (Fig. 5c);
- Fign. 6a-6c: das in Fig. 5 gezeigte eine, erste Clipteil, welches einstückig mit einer Lagerhülse gefertigt ist, in einer Draufsicht auf die Clipteil-Innenseite (Fig. 6a), in einer Schnittansicht (Fig. 6b) und in einer Draufsicht auf die Clipteil-Außenseite (Fig. 6c);
- Fign. 7a, 7b: das in Fig. 5 gezeigte andere, zweite Clipteil in einer Draufsicht auf die Clipteil-Innenseite (Fig. 7a) und in einer Seitenansicht (Fig. 7b);
- Fig. 8: die beiden Clipteile, die für einen Drehsteckschluss in einer maximal weit geöffneten Montagedrehposition ineinander gesteckt und über die Lagerhülse drehbar gelagert sind;
- Fign. 9a, 9b: eine zweite Ausführungsform des erfindungsgemäßen Aneurysma-Clips, der aus zwei Clipteilen und einer Lagerhülse zusammengesteckt ist, in geschlossenem Zustand in einer Schnittansicht (Fig. 9a) und in einer Draufsicht (Fig. 9b);
- Fign. 10a, 10b: das in Fig. 9 gezeigte eine, erste Clipteil in einer Schnittansicht (Fig. 10a) und in einer Draufsicht auf die Clipteil-Innenseite (Fig. 10b);
- Fign. 11a, 11b: die in Fig. 9 gezeigte Lagerhülse in einer Schnittansicht (Fig. 11a) und in einer Draufsicht (Fig. 11 b);
- Fig. 12: das in Fig. 10 gezeigte erste Clipteil mit der darin unverdrehbar eingesteckten Lagerhülse in einer Schnittansicht;
- Fign. 13a, 13b: das in Fig. 9 gezeigte andere, zweite Clipteil in einer Draufsicht auf die Clipteil-Innenseite (Fig. 13a) und in einer Schnittansicht (Fig. 13b);
- Fign. 14a, 14b: eine dritte Ausführungsform des erfindungsgemäßen Aneurysma-Clips, der aus zwei Clipteilen und einer Lagerhülse zusammengesteckt ist, in geschlossenem Zustand in einer Schnittansicht (Fig. 14a) und in einer Draufsicht (Fig. 14b);
- Fign. 15a, 15b: das in Fig. 14 gezeigte eine, erste Clipteil in einer Schnittansicht (Fig. 15a) und in einer Draufsicht auf die Clipteil-Innenseite (Fig. 15b);
- Fign. 16a, 16b: die in Fig. 14 gezeigte Lagerhülse in einer Schnittansicht (Fig. 16a) und in einer Draufsicht (Fig. 16b);
- Fig. 17: das in Fig. 15 gezeigte erste Clipteil mit der darin unverdrehbar eingesteckten Lagerhülse in einer Schnittansicht; und
- Fign. 18a, 18b: das in Fig. 14 gezeigte andere, zweite Clipteil in einer Draufsicht auf die Clipteil-Innenseite (Fig. 18a) und in einer Seitenansicht (Fig. 18b).

In der folgenden Beschreibung der Zeichnung werden für gleiche bzw. funktionsgleiche Bauteile identische Bezugszeichen verwendet.

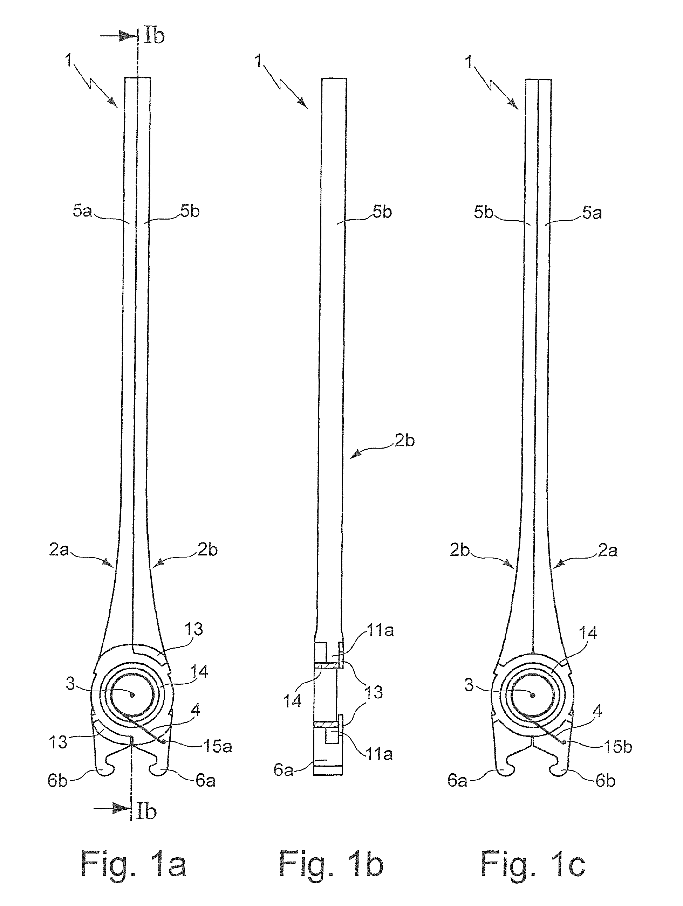

Der in **Fign. 1a-1c** gezeigte Aneurysma-Clip 1 umfasst zwei doppelarmige Clipteile **2a**, **2b**, die um eine Drehachse 3 drehbar miteinander verbunden und durch eine mindestens eine Windung aufweisende Schenkelfeder **4** in ihre geschlossene Endposition vorgespannt sind.

Die beiden Clipteile 2a, 2b weisen jeweils einen langen Klemmarm **5a**, **5b** und einen kurzen Bedienarm **6a**, **6b** auf, die bezüglich der Drehachse 3 einander gegenüber liegen und zueinander parallelversetzt sind. Mithilfe einer zwischen die beiden Bedienarme 6a, 6b greifenden Anlegezange können die Bedienarme 6a, 6b gegen die Schließkraft der Schenkelfeder 4 auseinandergedrückt und dadurch die Klemmarme 5a, 5b geöffnet werden.

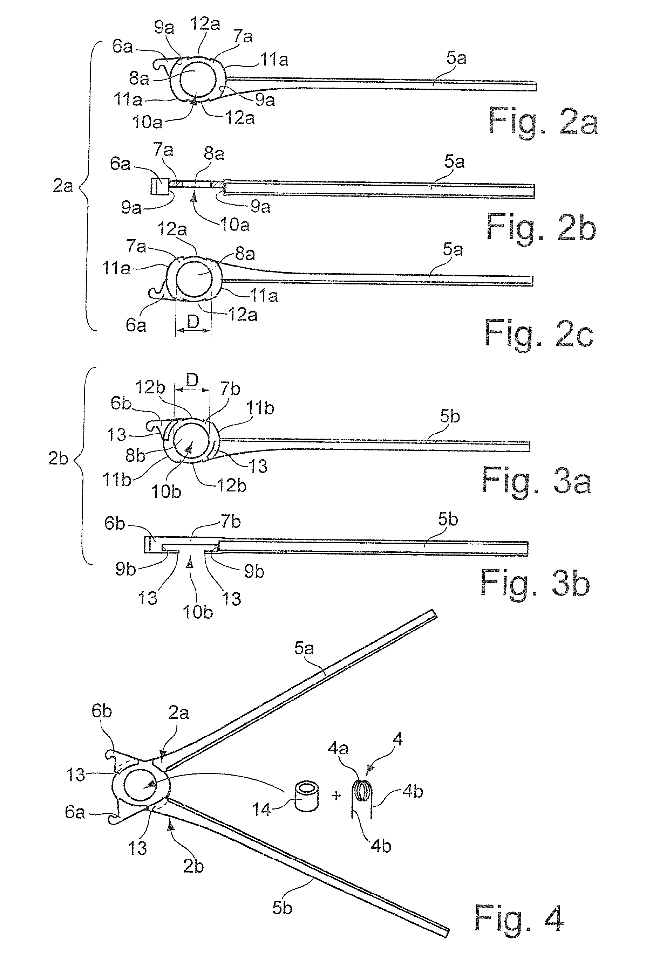

Wie in **Fign. 2a-2c** gezeigt ist, weist das eine, erste Clipteil 2a zwischen Klemm- und Bedienarm 5a, 6a einen flachen Ringabschnitt **7a** mit einer kreisrunden Öffnung **8a** (Öffnungsdurchmesser **D**) auf. Der Ringabschnitt 7a ist gegenüber den Klemm- und Bedienarmen 5a, 6a durch zwei teilzylinderförmige Absätze **9a** axial zurückversetzt, um dadurch eine axial offene Steckaufnahme **10a** auszubilden. Der Ringabschnitt 7a bildet den Boden bzw. die Bodenplatte der Steckaufnahme 10a, und die beiden Absätze 9a bilden zwei bezüglich der Öffnung 8a einander gegenüberliegende Seitenwände der Steckaufnahme 10a. Der Ringabschnitt 7a weist zwei bezüglich der Öffnung 8a einander gegenüberliegende erste Ringsegmente **11a** und dazwischen jeweils ein zweites Ringsegment **12a** auf, wobei die zweiten Ringsegmente 12a gegenüber den ersten Ringsegmenten 11a jeweils radial nach innen zurückversetzt sind.

Wie in **Fign. 3a, 3b** gezeigt ist, weist das andere, zweite Clipteil 2b zwischen Klemm- und Bedienarm 5b, 6b ebenfalls einen flachen Ringabschnitt **7b** mit einer kreisrunden Öffnung **8b** (Öffnungsdurchmesser **D**) auf. Der Ringabschnitt 7b ist gegenüber den Klemm- und Bedienarmen 5b, 6b durch zwei teilzylinderförmige Absätze **9b** axial zurückversetzt, um dadurch eine axial offene Steckaufnahme **10b** auszubilden. Der Ringabschnitt 7b bildet den Boden bzw. die Bodenplatte der Steckaufnahme 10b, und die beiden Absätze 9b bilden zwei bezüglich der Öffnung 8b einander gegenüberliegende Seitenwände der Steckaufnahme 10b. Der Ringabschnitt 7b weist zwei bezüglich der Öffnung 8b einander gegenüberliegende erste Ringsegmente **11b** und dazwischen jeweils ein zweites Ringsegment **12b** auf, wobei die zweiten Ringsegmente 12b gegenüber den ersten Ringsegmenten 11b jeweils radial nach innen zurückversetzt sind. Die beiden Absätze 9b sind auf der der Bodenplatte 7b gegenüberliegenden Seite jeweils von einem Überstand **13** übergriffen und dadurch als Umfangsnuten ausgebildet. Mit Ausnahme seiner beiden Überstände 13 kann das zweite Clipteil 2b identisch dem ersten Clipteil 2a ausgebildet sein. Die zweiten Ringsegmente 12b sind zwar funktionsmäßig nicht erforderlich, aber ermöglichen es, dass die beiden Clipteile 2a, 2b aus gleichen Rohteilen gefertigt werden können.

Zum Montieren des Aneurysma-Clips 1 werden das erste Clipteil 2a mit seinen zweiten Ringsegmenten 12a zwischen den beiden Überständen 13 des zweiten Clipteils 2b und das zweite Clipteil 2b mit seinen zweiten Ringsegmenten 12b zwischen den beiden Absätzen 9a des ersten Clipteils 2a ausgerichtet und in dieser in **Fig. 4** gezeigten maximal weit geöffneten Montagedrehposition axial ineinander gesteckt, bis sie mit ihren planen Bodenplatten 7a, 7b innenseitig aneinander anliegen und sich ihre Öffnungen 8a, 8b decken. Durch diese Öffnungen 8a, 8b wird dann eine Lagerhülse **14** gesteckt, deren runder Außendurchmesser bis auf ein minimales Lagerspiel dem Öffnungsdurchmesser D der kreisrunden Öffnungen 8a, 8b entspricht. Die Lagerhülse 14 bildet somit die Drehlagerung, um die sich die beiden Clipteile 2a, 2b drehen. Die Länge der Lagerhülse 14 entspricht maximal der Gesamtdicke der beiden Ringabschnitte 7a, 7b, so dass die Lagerhülse 14 bündig mit den Außenseiten der beiden Clipteile 2a, 2b abschließt bzw. zurückversetzt ist.

Anschließend werden für einen Drehsteckschluss die beiden Clipteile 2a, 2b in Richtung auf ihre geschlossene Endposition gedreht, wodurch die ersten Ringsegmente 11a des ersten Clipteils 2a in die Umfangsnuten 9b des ersten Clipteils 2a eingreifen und somit die beiden Clipteile 2a, 2b miteinander axial verbunden bzw. entgegen der Steckrichtung verriegelt sind.

Abschließend wird die Schenkelfeder 4 mit ihrem Federwindungskörper **4a** in die Lagerhülse 14 eingefädelt und dann mit ihren beiden Federschenkeln **4b** außenseitig bei **15a**, **15b** (Fig. 1) an den Bedienarmen 6a, 6b angeschweißt. Dadurch sind einerseits die beiden Clipteile 2a, 2b in ihre geschlossene Ausgangsdrehstellung vorgespannt und in der Montagedrehposition entgegen der Steckrichtung gesichert, und andererseits ist die Lagerhülse 14 zwischen den beiden Schenkeln 4b unverlierbar im Aneurysma-Clip 1 gehalten. Vorteilhaft ist die Länge der Lagerhülse 14 kleiner als die Gesamtdicke der beiden Ringabschnitte 7a, 7b, so dass keine Reibung zwischen der Lagerhülse 14 und den Schenkeln 4b stattfindet.

Statt mit den beiden Clipteilen 2a, 2b verschweißt können alternativ die beiden Federschenkel die beiden Bedienarme 6a, 6b - ähnlich einer Wäscheklammer - außenseitig umklammern. In diesem Fall können die beiden Clipteilen 2a, 2b auch aus nicht-schweißbarem Material, wie z.B. aus Kunststoff, insbesondere aus Polymethylmethacrylat (PMMA) oder aus röntgentransparentem Polyetheretherketon (PEEK), gebildet sein.

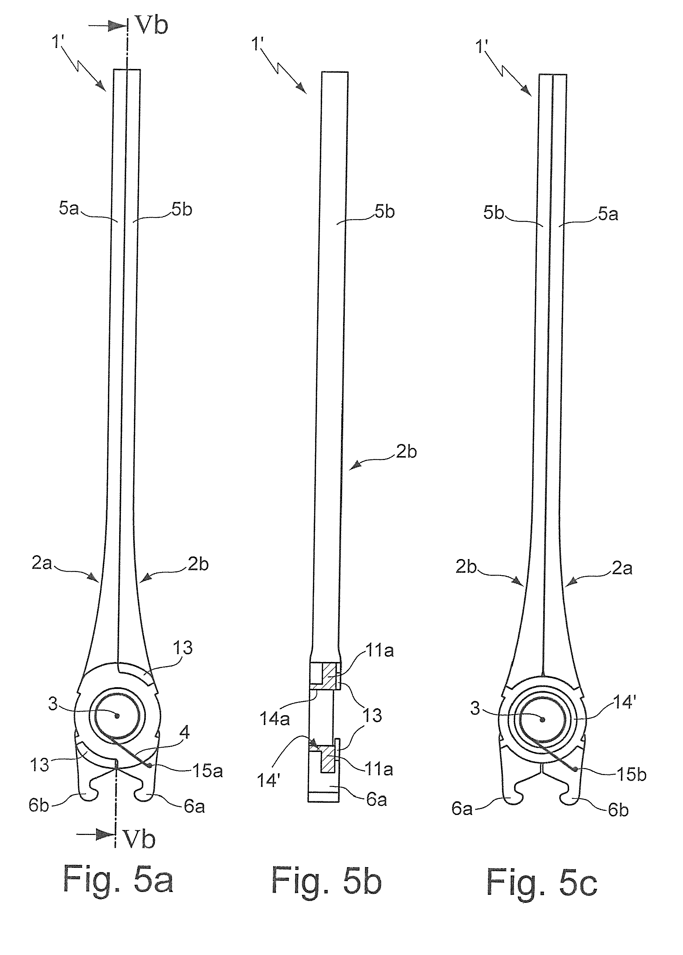

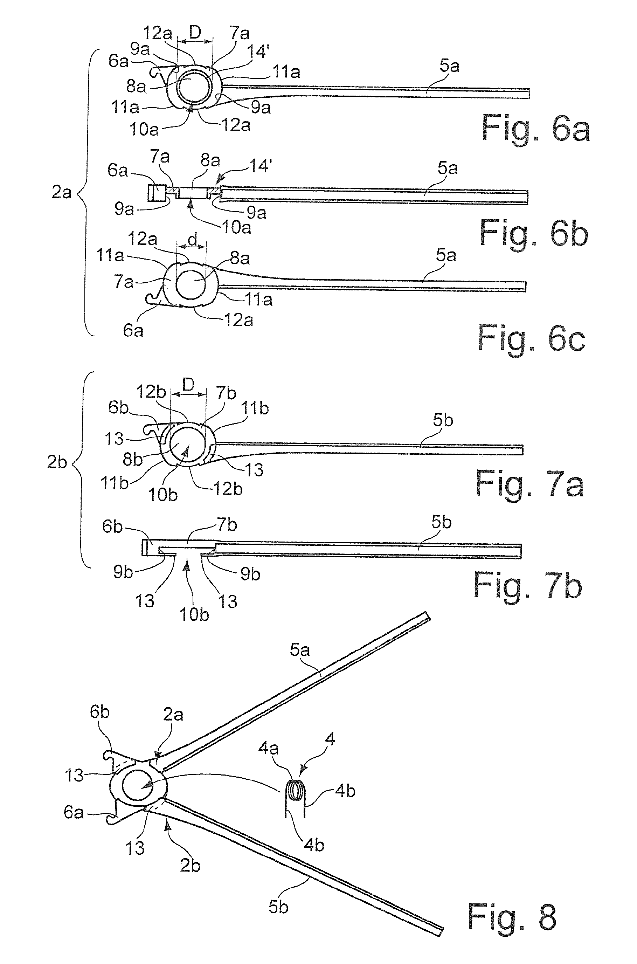

Vom Aneurysma-Clip 1 der Fig. 1 unterscheidet sich der in **Fig.** 5 gezeigte Aneurysma-Clip **1'** einzig dadurch, dass hier die Lagerhülse 14' einstückig mit dem ersten Clipteil 2a gefertigt ist. Wie in **Fign. 6a-6c** gezeigt ist, steht die Lagerhülse 14' (Außendurchmesser **D**) axial über den Ringabschnitt 7a vor. Die Hülsenöffnung (Innendurchmesser **d**) der Lagerhülse 14' erstreckt sich durchgehend bis zur Clipteil-Außenseite und definiert somit die Öffnung 8a des Ringabschnitts 7a. Das in **Fign. 7a**, **7b** gezeigte zweite Clipteil 2b des Aneurysma-Clips 1' ist identisch wie das Clipteil 2b der Fign. 3a, 3b ausgebildet.

Zum Montieren des Aneurysma-Clips 1' werden das erste Clipteil 2a mit seinen zweiten Ringsegmenten 12a zwischen den beiden Überständen 13 des zweiten Clipteils 2b und das zweite Clipteil 2b mit seinen zweiten Ringsegmenten 12b zwischen den beiden Absätzen 9a des ersten Clipteils 2a ausgerichtet und in dieser in **Fig. 8** gezeigten maximal weit geöffneten Montagedrehposition axial ineinander gesteckt, bis das erste Clipteil 2a mit seiner Lagerhülse 14' in die Öffnung 8b des zweiten Clipteils 2b eingesteckt ist und die beiden planen Bodenplatten 7a, 7b innenseitig aneinander anliegen. Die Lagerhülse 14' bildet somit die Drehlagerung, um die sich die beiden Clipteile 2a, 2b drehen. Die vorstehende Länge der Lagerhülse 14' entspricht maximal der Dicke des Ringabschnitts 7b des zweiten Clipteils 2b, so dass die Lagerhülse 14' bündig mit der Außenseite des zweiten Clipteils 2b abschließt oder axial zurückversetzt ist. Vorteilhaft ist die Länge der Lagerhülse 14' kleiner als die Dicke des Ringabschnitts 7b, so dass keine Reibung zwischen der Lagerhülse 14' und dem Schenkeln 4b stattfindet.

Anschließend werden für einen Drehsteckschluss die beiden Clipteile 2a, 2b in Richtung auf ihre geschlossene Endposition gedreht, wodurch die ersten Ringsegmente 11a des ersten Clipteils 2a in die Umfangsnuten 9b des zweiten Clipteils 2b eingreifen und somit die beiden Clipteile 2a, 2b miteinander axial verbunden bzw. entgegen der Steckrichtung verriegelt sind.

Abschließend wird die Schenkelfeder 4 mit ihrem Federwindungskörper 4a in die Hülsenöffnung 8a der Lagerhülse 14' eingefädelt und dann mit ihren beiden Schenkeln 4b außenseitig bei 15a, 15b (Fig. 5) an den Bedienarmen 6a, 6b angeschweißt. Dadurch sind die beiden Clipteile 2a, 2b in ihre geschlossene Ausgangsdrehstellung vorgespannt und in der Montagedrehposition entgegen der Steckrichtung gesichert.

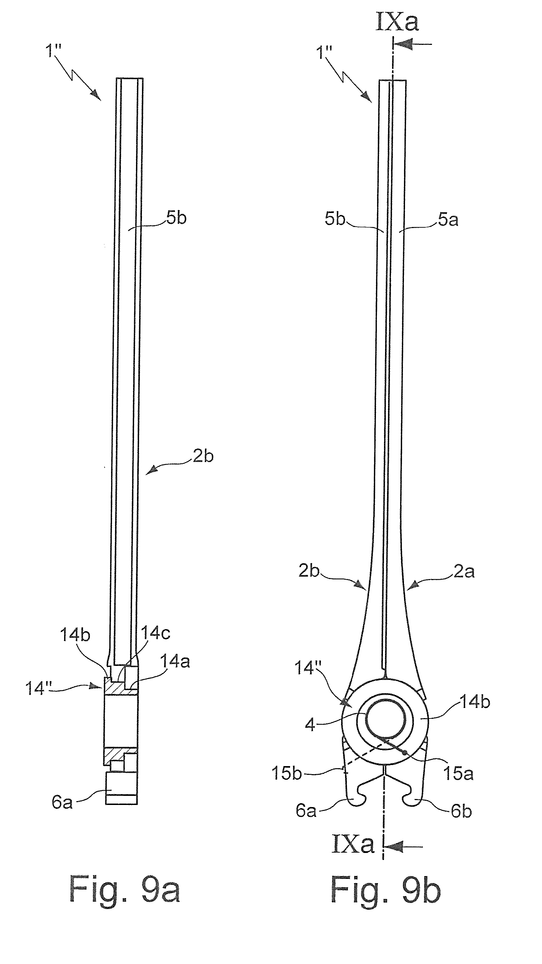

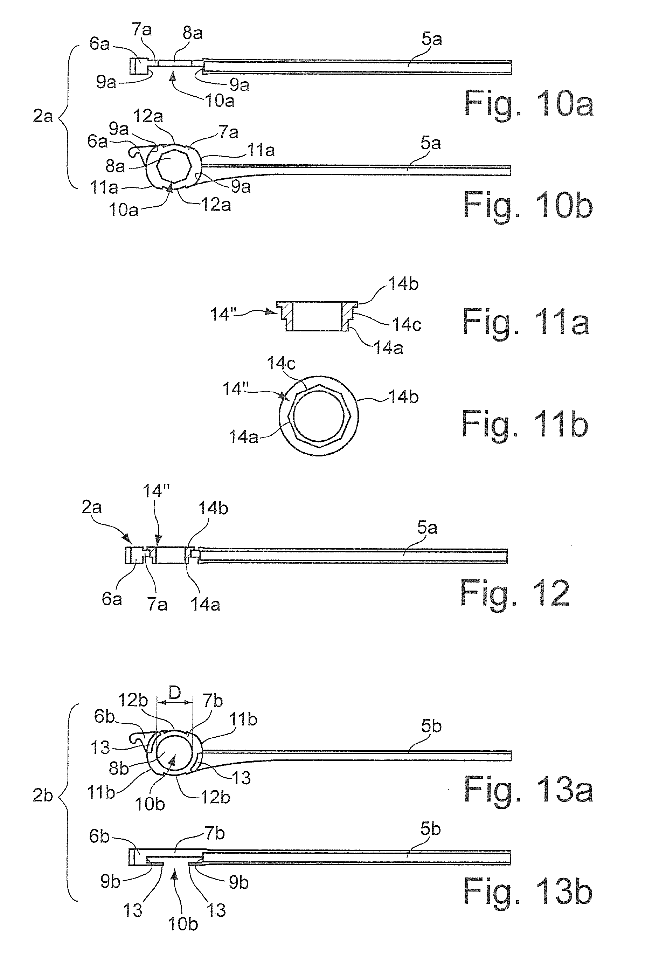

Vom Aneurysma-Clip 1 der Fig. 1 unterscheidet sich der in **Fign. 9a**, **9b** gezeigte Aneurysma-Clip **1"** einzig dadurch, dass hier die Lagerhülse 14" unverdrehbar in das erste Clipteil 2a eingesteckt ist. Das in **Fign. 10a, 10b** gezeigte erste Clipteil 2a des Aneurysma-Clips 1" weist eine Öffnung 8a mit achteckigen Öffnungsquerschnitt auf, und die in **Fign. 11a, 11b** gezeigte Lagerhülse 14" weist einenends einen Lagerabschnitt **14a** mit kreisrundem Außenquerschnitt, andernends einen Ringbund 14b sowie dazwischen einen Steckabschnitt 14c mit achteckigem Außenquerschnitt auf, wobei der Steckabschnitt 14c gegenüber dem Ringbund 14b und der Lagerabschnitt 14a gegenüber dem Steckabschnitt 14c jeweils radial nach innen zurückversetzt sind. Wie in **Fig**. **12** gezeigt, ist die Lagerhülse 14" mit ihrem achteckigen Steckabschnitt 14c in die achteckige Öffnung 8a des ersten Clipteils 2a eingesteckt und somit darin unverdrehbar gehalten. Die Lagerhülse 14" liegt mit ihrem Ringbund 14b außenseitig am Ringabschnitt 7a des ersten Clipteils 2a an und steht mit ihrem kreisrunden Lagerabschnitt 14a innenseitig über den Ringabschnitt 7a vor. Statt wie gezeigt achteckig, können der Öffnungsquerschnitt der Öffnung 8a und der Außenquerschnitt des Steckabschnitts 14c auch jeden anderen nicht-kreisrunden Querschnitt aufweisen, z.B. einen anderen polygonen oder einen ovalen Querschnitt. Das in **Fign. 13a, 13b** gezeigte zweite Clipteil 2b des Aneurysma-Clips 1" ist identisch wie das Clipteil 2b der Fign. 3a, 3b ausgebildet.

Das erste Clipteil 2a mit der eingesteckten Lagerhülse 14" und das zweite Clipteil 2b werden wie der in Fig. 5 gezeigte Aneurysma-Clips 1' montiert. Die Lagerhülse 14" bildet somit die Drehlagerung, um die sich das zweite Clipteile 2b dreht. Die vorstehende Länge des Lagerabschnitts 14b der Lagerhülse 14" entspricht maximal der Dicke des Ringabschnitts 7b des zweiten Clipteils 2b, so dass die Lagerhülse 14" entweder bündig mit der Außenseite des zweiten Clipteils 2b abschließt oder axial zurückversetzt ist. Abschließend wird die Schenkelfeder 4 mit ihrem Federwindungskörper in die Hülsenöffnung der Lagerhülse 14" eingefädelt und dann mit ihren beiden Federschenkeln an den beiden Clipteilen 2a, 2b befestigt, um die beiden Clipteile 2a, 2b in die geschlossene Endposition vorzuspannen. Im gezeigten Ausführungsbespiel ist die Schenkelfeder 4 mit ihrem einen Federschenkel bei 15a an die Lagerhülse 14" angeschweißt, und zwar vorteilhaft bereits vor dem Einstecken der Lagerhülse 14" in das erste Clipteil 2a, und umgreift mit ihrem anderen Federschenkel - ähnlich einer Wäscheklammer - bei 15b den Bedienarm 6b des zweiten Clipteils 2b. Die Schenkelfeder 4 ist also nicht mit den Clipteilen 2a, 2b verschweißt, so dass die Clipteile 2a, 2b auch aus nicht-schweißbarem Material, wie z.B. aus Kunststoff, insbesondere aus Polymethylmethacrylat (PMMA) oder aus röntgentransparentem Polyetheretherketon (PEEK), gebildet sein können. Vorteilhaft ist die Länge des Lagerabschnitts 14b kleiner als die Dicke des Ringabschnitts 7b, so dass keine Reibung zwischen der Lagerhülse 14" und dem Schenkeln 4b stattfindet.

Sofern die Clipteile 2a, 2b aus schweißbarem Material gebildet sind, kann die Schenkelfeder 4 - wie bei den Aneurysmenclips 1, 1' - am Bedienarm 6b des zweiten Clipteils 2b und optional statt an der Lagerhülse 4" auch am Bedienarm 6a des ersten Clipteils 2a angeschweißt sein.

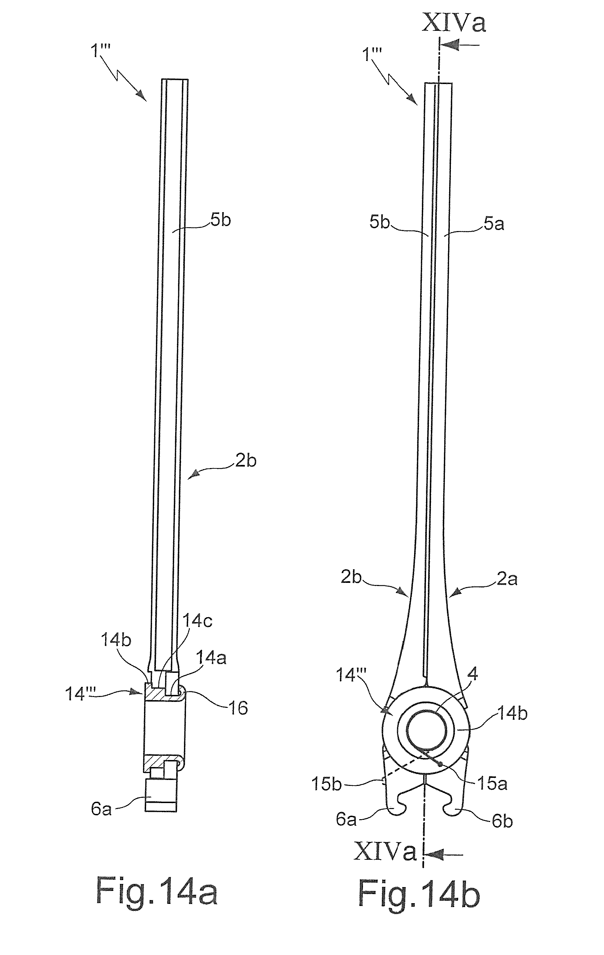

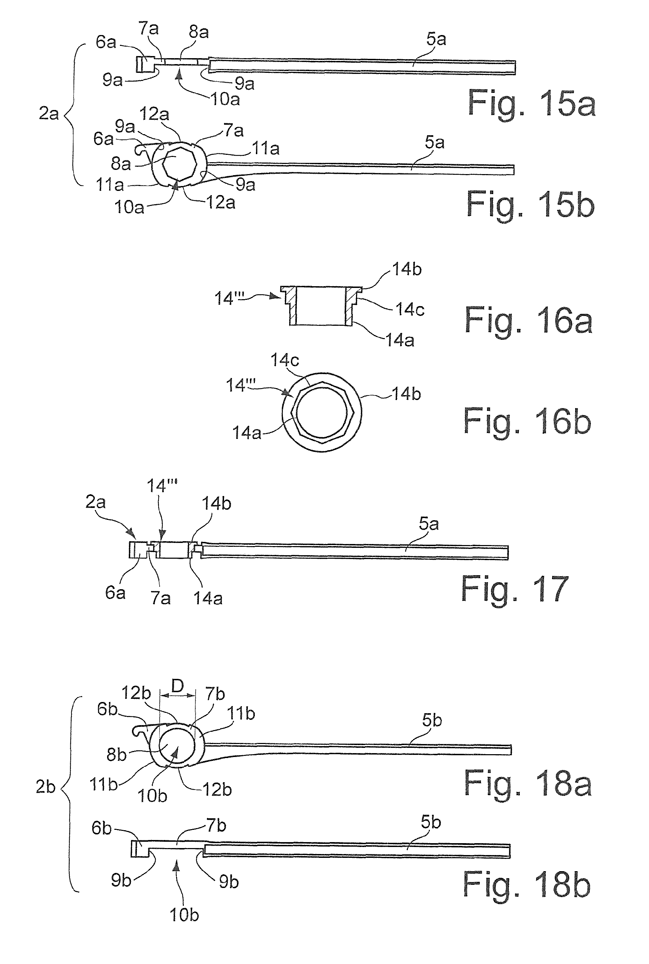

Vom Aneurysma-Clip 1" der Fig. 9 unterscheidet sich der in **Fign. 14a, 14b** gezeigte Aneurysma-Clip **1'"** einzig dadurch, dass die beiden Clipteile 2a, 2b nicht durch einen Drehsteckschluss miteinander verbunden sind, sondern durch Umbiegen der Lagerhülse **14"'**. Das in **Fign. 15a**, **15b** gezeigte erste Clipteil 2a des Aneurysma-Clips 1'" ist identisch wie das Clipteil 2a der Fign. 10a, 10b ausgebildet. Die in **Fign. 16a**, **16b** gezeigte Lagerhülse 14"' unterscheidet sich von der in Fig. 11 gezeigten Lagerhülse 14" lediglich durch einen längeren Lagerabschnitt 14a. **Fig. 17** zeigt das erste Clipteil 2a mit der darin unverdrehbar eingesteckten Lagerhülse 14"'. Das in **Fign. 18a, 18b** gezeigte zweite Clipteil 2b des Aneurysma-Clips 1"' ist bis auf die fehlenden Überstande 13 identisch wie das Clipteil 2b der Fign. 3a, 3b ausgebildet.

Das erste Clipteil 2a mit der eingesteckten Lagerhülse 14'" und das zweite Clipteil 2b werden wie der in Fig. 9 gezeigte Aneurysma-Clips 1" montiert. Die Lagerhülse 14'" bildet somit die Drehlagerung, um die sich das zweite Clipteile 2b dreht. Das über die Außenseite des zweiten Clipteils 2b überstehende Ende des längeren Lagerabschnitts 14b der Lagerhülse 14"' wird nach außen zu einem Nietkopf 16 umgebogen, um die beiden Clipteile 2a, 2b aneinander zu befestigen bzw. axial zusammenzuhalten.

## Patentansprüche

1. Chirurgischer Clip (1), insbesondere Aneurysma-Clip, mit zwei drehbar miteinander verbundenen Clipteilen (2a, 2b), welche jeweils einen Klemmarm (5a, 5b), einen Bedienarm (6a, 6b) und einen dazwischen befindlichen Ringabschnitt (7a, 7b) mit einer Öffnung (8a, 8b) aufweisen, und mit einer Schenkelfeder (4), welche die beiden Clipteile (2a, 2b) in eine Ausgangsdrehstellung vorspannt, wobei die Drehlagerung der beiden Clipteile (2a, 2b) durch eine Lagerhülse (14) gebildet ist, wobei die Schenkelfeder (4) mit ihrem Windungskörper (4a) zumindest teilweise innerhalb der Lagerhülse (14) angeordnet ist und wobei die Öffnungen (8a, 8b) der beiden Clipteile (2a, 2b) kreisrund ausgebildet sind,
**dadurch gekennzeichnet,**
**dass** die Lagerhülse (14) ein separates Teil ist, das in beide Öffnungen (8a, 8b) gesteckt und darin jeweils drehbar gelagert ist.

2. Chirurgischer Clip nach Anspruch 1, **dadurch gekennzeichnet, dass** die kreisrunden Öffnungen (8a, 8b) der beiden Clipteile (2a, 2b) den gleichen Öffnungsdurchmesser (D) aufweisen.

3. Chirurgischer Clip (1"; 1"'), insbesondere Aneurysma-Clip, mit zwei drehbar miteinander verbundenen Clipteilen (2a, 2b), welche jeweils einen Klemmarm (5a, 5b), einen Bedienarm (6a, 6b) und einen dazwischen befindlichen Ringabschnitt (7a, 7b) mit einer Öffnung (8a, 8b) aufweisen, und mit einer Schenkelfeder (4), welche die beiden Clipteile (2a, 2b) in eine Ausgangsdrehstellung vorspannt, wobei die Drehlagerung der beiden Clipteile (2a, 2b) durch eine Lagerhülse (14"; 14"') gebildet ist, wobei die Schenkelfeder (4) mit ihrem Windungskörper (4a) zumindest teilweise innerhalb der Lagerhülse (14"; 14"') angeordnet ist und wobei die Lagerhülse (14"; 14"') an dem einen Clipteil (2a) unverdrehbar befestigt ist und einen Lagerabschnitt (14a) aufweist, der in die kreisrund ausgebildete Öffnung (8b) des anderen Clipteils (2b) gesteckt und darin drehbar gelagert ist,
**dadurch gekennzeichnet,**
**dass** die Lagerhülse (14"; 14"') einen Steckabschnitt (14c) mit nichtkreisförmigem Außenquerschnitt aufweist, der in die nicht-kreisrund ausgebildete Öffnung (8a) des einen Clipteils (2a) unverdrehbar eingesteckt ist.

4. Chirurgischer Clip nach Anspruch 3, **dadurch gekennzeichnet, dass** die beiden Clipteile (2a, 2b) aus schweißbarem Material gebildet sind und dass die beiden Federschenkel der Schenkelfeder (4) mit den beiden Clipteilen (2a, 2b) verschweißt sind oder mit der drehfest an dem einen Clipteil (2a) befestigten Lagerhülse (14"; 14"') und mit dem anderen Clipteil (2b) verschweißt sind.

5. Chirurgischer Clip nach Anspruch 3, **dadurch gekennzeichnet, dass** die beiden Clipteile (2a, 2b) aus nicht-schweißbarem Material gebildet sind und dass der eine Federschenkel der Schenkelfeder (4) mit der drehfest an dem einen Clipteil (2a) befestigten Lagerhülse (14"; 14"') verschweißt ist und der andere Federschenkel am anderen Clipteil (2b) angreift.

6. Chirurgischer Clip nach einem der vorhergehenden Ansprüche, **dadurch gekennzeichnet, dass** der Windungskörper (4a) der Schenkelfeder (4) vollständig innerhalb der Lagerhülse (14; 14"; 14'") angeordnet ist.

7. Chirurgischer Clip nach einem der vorhergehenden Ansprüche, **dadurch gekennzeichnet, dass** die beiden Clipteile (2a, 2b) durch einen Steck-Drehverschluss miteinander verbunden sind, bei dem die beiden Clipteile (2a, 2b) in einer Montagedrehposition axial ineinander gesteckt und durch anschließendes Verdrehen in Richtung auf die Ausgangsdrehstellung miteinander axial verriegelt sind.

8. Chirurgischer Clip nach Anspruch 7, **dadurch gekennzeichnet, dass** der Ringabschnitt (7a) mindestens eines der beiden Clipteile (2a, 2b) zwei bezüglich der Öffnung (8a) einander gegenüberliegende erste Ringsegmente (11a) und dazwischen jeweils ein gegenüber den ersten Ringsegmenten (11a) radial nach innen zurückversetztes zweites Ringsegment (12a, 12b) aufweist und dass mindestens das andere Clipteil (2b) eine Steckaufnahme (10b) aufweist, die durch zwei bezüglich der Öffnung (8b) einander gegenüberliegende Seitenwände (9b) mit darin vorgesehen Umfangsnuten (9b) gebildet ist, wobei in der Montagedrehposition das eine Clipteil (2a) mit seinen beiden zweiten Ringsegmenten (12a) zwischen die Seitenwände (9b) des jeweils anderen Clipteils (2b) in die Steckaufnahme (10b) gesteckt und durch anschließendes Verdrehen mit seinen ersten Ringsegmenten (11a,) in die Umfangsnuten (9b) des anderen Clipteils (2b) eingreift und dadurch die beiden Clipteile (2a, 2b) miteinander entgegen der Steckrichtung verriegelt sind.

9. Chirurgischer Clip nach einem der vorhergehenden Ansprüche, **dadurch gekennzeichnet, dass** die beiden Clipteile (2a, 2b) durch die Lagerhülse (14"'), deren eines Ende einen Ringbund (14b) und deren anderes Ende einen nach außen umgebogenen Nietkopf (16) aufweist, axial zusammengehalten sind.

10. Chirurgischer Clip nach einem der vorhergehenden Ansprüche, **dadurch gekennzeichnet, dass** die beiden Clipteile (2a, 2b) mit den einander zugewandten ebenen Innenflächen ihrer Ringabschnitte (7a, 7b) aufeinanderliegen.

## Claims

1. Surgical clip (1), in particular aneurysm clip, comprising two rotatably connected clip parts (2a, 2b), each having a clamping arm (5a, 5b), an operating arm (6a, 6b), and an interposed annular section (7a, 7b) with an opening (8a, 8b), and comprising a leg spring (4) which pretensions the two clip parts (2a, 2b) into an initial rotation position, wherein the pivot bearing of the two clip parts (2a, 2b) is formed by a bearing sleeve (14), wherein the winding body (4a) of the leg spring (4) is arranged at least in part inside the bearing sleeve (14) and wherein the openings (8a, 8b) of the two clip parts (2a, 2b) are circular,
**characterized in that**
the bearing sleeve (14) is a separate part which is fitted into both openings (8a, 8b) and is pivoted therein.

2. Surgical clip according to claim 1, **characterized in that** the circular openings (8a, 8b) of the two clip parts (2a, 2b) have the same opening diameter (D).

3. Surgical clip (1"; 1"'), in particular aneurysm clip, comprising two rotatably connected clip parts (2a, 2b), each having a clamping arm (5a, 5b), an operating arm (6a, 6b), and an interposed annular section (7a, 7b) with an opening (8a, 8b), and comprising a leg spring (4) which pretensions the two clip parts (2a, 2b) into an initial rotation position, wherein the pivot bearing of the two clip parts (2a, 2b) is formed by a bearing sleeve (14"; 14"'), wherein the winding body (4a) of the leg spring (4) is arranged at least in part inside the bearing sleeve (14"; 14"') and wherein the bearing sleeve (14"; 14"') is non-rotatably mounted to one clip part (2a) and has a bearing section (14a) which is fitted into the circular opening (8b) of the other clip part (2b) and is pivoted therein,
**characterized in that**
the bearing sleeve (14"; 14"') has a fitting section (14c) of non-circular outer cross-section which is non-rotatably fitted into the non-circular opening (8a) of one (2a) of the clip parts.

4. Surgical clip according to claim 3, **characterized in that** the two clip parts (2a, 2b) are formed from weldable material and the two spring legs of the leg spring (4) are welded to the two clip parts (2a, 2b) or are welded to both the bearing sleeve (14"; 14"') which is non-rotatably mounted to one (2a) of the clip parts, and to the other clip part (2b).

5. Surgical clip according to claim 3, **characterized in that** the two clip parts (2a, 2b) are formed from non-weldable material and one spring leg of the leg spring (4) is welded to the bearing sleeve (14"; 14"') which is non-rotatably mounted to one (2a) of the clip parts, and the other spring leg engages the other clip part (2b).

6. Surgical clip according to any one of the preceding claims, **characterized in that** the winding body (4a) of the leg spring (4) is arranged completely inside the bearing sleeve (14; 14"; 14"').

7. Surgical clip according to any one of the preceding claims, **characterized in that** the two clip parts (2a, 2b) are connected to each other via a push-fit rotary lock, wherein the two clip parts (2a, 2b) are axially fitted into one another in an assembly rotation position and are axially locked to each other by subsequent rotation towards the initial rotation position.

8. Surgical clip according to claim 7, **characterized in that** the annular section (7a) of at least one of the two clip parts (2a, 2b) has two first annular segments (11 a) which are disposed opposite to each other with respect to the opening (8a), and a respectively interposed second annular segment (12a, 12b) which is radially set back towards the inside with respect to the first annular segments (11a), and at least the other clip part (2b) has a push-fit receptacle (10b) which is formed by two side walls (9b) which are disposed opposite to each other with respect to the opening (8b) and are provided with circumferential grooves (9b), wherein in the assembly rotation position, the two second annular segments (12a) of one (2a) of the clip parts are fitted into the push-fit receptacle (10b) between the side walls (9b) of the respective other clip part (2b) and through subsequent rotation, the first annular segments (11a) of the one clip part (2a) engage the circumferential grooves (9b) of the other clip part (2b), thereby locking the two clip parts (2a, 2b) to each other in a direction opposite to the fitting direction.

9. Surgical clip according to any one of the preceding claims, **characterized in that** the two clip parts (2a, 2b) are axially held together by the bearing sleeve (14"'), one end of which has an annular flange (14b) and the other end of which has a rivet head (16) that is bent to the outside.

10. Surgical clip according to any one of the preceding claims, **characterized in that** the flat inner surfaces of the annular sections (7a, 7b) of the two clip parts (2a, 2b), which face each other, lie on top of each other.

## Revendications

1. Clip chirurgical (1), en particulier clip d'anévrisme, avec deux parties de clip (2a, 2b) reliées entre elles à rotation, qui présentent chacune un bras de serrage (5a, 5b), un bras de commande (6a, 6b) et entre ces bras une partie annulaire (7a, 7b) ayant une ouverture (8a, 8b), et avec un ressort à branches (4) qui précontraint les deux parties de clip (2a, 2b) dans une position de rotation initiale, sachant que le montage à rotation des deux parties de clip (2a, 2b) est formé par une douille de palier (14), sachant que le ressort à branches (4) est disposé au moins partiellement à l'intérieur de la douille de palier (14) par son corps enroulé (4a) et sachant que les ouvertures (8a, 8b) des deux parties de clip (2a, 2b) sont réalisées circulaires,
**caractérisé en ce que** la douille de palier (14) est une pièce séparée, qui est emboîtée dans les deux ouvertures (8a, 8b) et y est respectivement montée à rotation.

2. Clip chirurgical selon la revendication 1, **caractérisé en ce que** les ouvertures circulaires (8a, 8b) des deux parties de clip (2a, 2b) présentent le même diamètre d'ouverture (D).

3. Clip chirurgical (1" ; 1"'), en particulier clip d'anévrisme, avec deux parties de clip (2a, 2b) reliées entre elles à rotation, qui présentent chacune un bras de serrage (5a, 5b), un bras de commande (6a, 6b) et entre ces bras une partie annulaire (7a, 7b) ayant une ouverture (8a, 8b), et avec un ressort à branches (4) qui précontraint les deux parties de clip (2a, 2b) dans une position de rotation initiale, sachant que le montage à rotation des deux parties de clip (2a, 2b) est formé par une douille de palier (14" ; 14"'), sachant que le ressort à branches (4) est disposé au moins partiellement à l'intérieur de la douille de palier (14" ; 14"') par son corps enroulé (4a) et sachant que la douille de palier (14" ; 14"') est fixée sans possibilité de rotation sur une première partie de clip (2a) et présente une partie formant palier (14a) qui est emboîtée dans l'ouverture réalisée circulaire (8b) de l'autre partie de clip (2b) et y est montée à rotation,
**caractérisé en ce que** la douille de palier (14" ; 14"') présente une partie d'emboîtement (14c) ayant une section extérieure non circulaire, qui est emboîtée sans possibilité de rotation dans l'ouverture réalisée non circulaire (8a) de la première partie de clip (2a).

4. Clip chirurgical selon la revendication 3, **caractérisé en ce que** les deux parties de clip (2a, 2b) sont constituées d'un matériau pouvant être soudé, et **en ce que** les deux branches de ressort du ressort à branches (4) sont soudées aux deux parties de clip (2a, 2b), ou sont soudées à la douille de palier (14" ; 14"') fixée en solidarité de rotation sur la première partie de clip (2a) et à l'autre partie de clip (2b).

5. Clip chirurgical selon la revendication 3, **caractérisé en ce que** les deux parties de clip (2a, 2b) sont constituées d'un matériau ne pouvant pas être soudé, et **en ce que** la première branche de ressort du ressort à branches (4) est soudée à la douille de palier (14" ; 14"') fixée en solidarité de rotation sur la première partie de clip (2a), et l'autre branche de ressort agit sur l'autre partie de clip (2b).

6. Clip chirurgical selon l'une des revendications précédentes, **caractérisé en ce que** le corps enroulé (4a) du ressort à branches (4) est disposé en totalité à l'intérieur de la douille de palier (14 ; 14" ; 14"').

7. Clip chirurgical selon l'une des revendications précédentes, **caractérisé en ce que** les deux parties de clip (2a, 2b) sont reliées entre elles par une fermeture tournante à emboîtement, selon laquelle les deux parties de clip (2a, 2b) sont axialement emboîtées l'une dans l'autre dans une position de rotation de montage, puis sont axialement verrouillées entre elles par rotation en direction de la position de rotation initiale.

8. Clip chirurgical selon la revendication 7, **caractérisé en ce que** la partie annulaire (7a) d'au moins une des deux parties de clip (2a, 2b) présente deux premiers segments d'anneau (11a) se faisant face par rapport à l'ouverture (8a) et, entre ceux-ci, un deuxième segment d'anneau respectif (12a, 12b) en retrait radialement vers l'intérieur par rapport aux premiers segments d'anneau (11a), et **en ce qu'**au moins l'autre partie de clip (2b) présente un logement d'emboîtement (10b) qui est formé par deux parois latérales (9b) se faisant face par rapport à l'ouverture (8b) et dans lesquelles sont prévues des rainures périphériques (9b), sachant que, dans la position de rotation de montage, la première partie de clip (2a) est, par ses deux deuxièmes segments d'anneau (12a), emboîtée dans le logement d'emboîtement (10b) entre les parois latérales (9b) de l'autre partie de clip respective (2b) puis, par rotation, s'engage par ses premiers segments d'anneau (11a) dans les rainures périphériques (9b) de l'autre partie de clip (2b), de sorte que les deux parties de clip (2a, 2b) sont verrouillées entre elles à l'encontre de la direction d'emboîtement.

9. Clip chirurgical selon l'une des revendications précédentes, **caractérisé en ce que** les deux parties de clip (2a, 2b) sont axialement solidarisées par la douille de palier (14"'), dont une extrémité présente un collet annulaire (14b) et l'autre extrémité une tête de rivet (16) recourbée vers l'extérieur.

10. Clip chirurgical selon l'une des revendications précédentes, **caractérisé en ce que** les deux parties de clip (2a, 2b) reposent l'une sur l'autre par les faces intérieures planes, tournées l'une vers l'autre, de leurs parties annulaires (7a, 7b).
